# FASCICULE DE BREVET EUROPEEN

(11) **EP 3 282 983 B1**
(45) Date de publication et mention de la délivrance du brevet: **04.05.2022**
(21) Numéro de dépôt: 16730876.6
(22) Date de dépôt: 14.04.2016
(51) Int. Cl.: A61B 17/70

(54) **DISPOSITIF ET SYSTEME DE FIXATION D'UNE VERTEBRE RACHIDIENNE SUR UNE TIGE**
VORRICHTUNG UND SYSTEM ZUR FIXIERUNG EINES RÜCKENWIRBELS AN EINEN STAB
DEVICE AND SYSTEM FOR FIXING A SPINAL VERTEBRA TO A ROD

(30) Priorité: 17.04.2015 FR 1553428
(43) Date de publication de la demande: 21.02.2018
(73) Titulaire: Implanet, 33650 Martillac (FR)
(72) Inventeur: LE COUËDIC, Régis, 33000 Bordeaux (FR); PASQUET, Denis, 13100 Aix en Provence (FR)
(74) Mandataire: Benech, Frédéric
(86) Numéro de dépôt international: PCT/FR2016/050867
(87) Numéro de publication internationale: WO 2016/166482

(56) Documents cités:
- EP-A1- 2 422 728
- EP-A1- 2 762 095
- WO-A1-2009/144663
- US-A- 5 383 905
- US-A1- 2010 094 345
- US-A1- 2011 245 875

## Description

La présente invention concerne un dispositif de fixation d'une vertèbre rachidienne sur une tige comportant une vis pédiculaire et une attache de fixation de la vis sur la tige.

Elle concerne également un système muni d'une tige et d'au moins un tel dispositif.

Un procédé de fixation d'une tige sur une vertèbre dans lequel on visse une vis pédiculaire dans la vertèbre, on glisse la tige au travers d'une attache de fixation de la vis sur la tige et on visse une vis de pression sur la tige au travers de la partie supérieure de l' attache est également décrit mais non revendiqué.

Elle trouve une application particulièrement importante bien que non exclusive dans le domaine du redressement de la colonne vertébrale présentant une courbure anormale.

Afin de redresser l'ensemble il est connu de rapprocher les bords latéraux des vertèbres de part et d'autre de la colonne vertébrale par le biais d'une tige reliant entre elles soit des vis, que l'on insère dans les vertèbres elles-mêmes, soit des crochets que l'on introduit le long du canal rachidien.

Ces dispositifs ne sont néanmoins pas entièrement satisfaisants.

En effet il peut advenir que l'os de la vertèbre ne soit pas de bonne qualité par exemple à cause d'ostéoporose.

En cas d'utilisation d'une vis, celle-ci doit alors être plus longue pour améliorer la fixation. Dans ce cas le chirurgien n'est cependant pas certain que le pédicule ne dépasse de la vertèbre une fois vissé.

De même dans le cas de la mise en œuvre de crochets, celle-ci est délicate et comporte un risque d'accident pouvant entrainer la paralysie du patient.

Une autre façon de gérer une mauvaise de qualité de l'os d'une vertèbre consiste à reporter la fixation sur la vertèbre d'à côté de meilleure qualité. Mais cela implique la fusion des vertèbres entre elles, ce qui favorise le syndrome dit « de charnière », l'ensemble des contraintes étant reporté au-dessus de l'empilage de vertèbres fusionnées entre elles.

Pour pallier ces inconvénients il a été proposé (FR 2 954 905, EP 2 762 095)) des systèmes mettant en œuvre un lien flexible de fixation de la vertèbre sur une pièce de liaison elle-même fixée à la tige.

De tels systèmes, permettant d'obtenir un serrage progressif et efficace, peuvent néanmoins entrainer dans certains cas, notamment lorsque la tige est en matière non métallique, un relâchement de la tension de la bande souple avec le temps.

Ils ne permettent pas non plus d'éviter le vrillage de la bande.

La présente invention vise à pallier les inconvénients de l'art antérieur.

Pour ce faire elle part de l'idée d'utiliser simultanément une vis pédiculaire et un lien flexible de fixation de la vertèbre sur une pièce fixée sur la tête de la vis pédiculaire.

La longueur de la vis peut ainsi être maintenue à une taille raisonnable même en cas de mauvaise qualité de l'os.

La présente invention vise donc à fournir un dispositif, un système de fixation d'une vertèbre rachidienne sur une tige répondant mieux que ceux antérieurement connus aux exigences de la pratique, notamment en ce qu'elle va permettre une excellente fixation de l'attache et ce pour un coût maîtrisé avec une grande souplesse et une meilleure flexibilité de montage par le chirurgien.

Dans ce but l'invention propose notamment un dispositif de fixation d'une vertèbre rachidienne sur une tige, comportant une vis pédiculaire, une attache de fixation de la vis sur la tige, et une bande souple de liaison avec la vertèbre, caractérisé en ce qu'il comporte une bague rapportée solidaire de l'attache , et des moyens solidaires de ladite bague, de blocage réglable de la bande souple par rapport à l'attache de fixation.

Avantageusement l'attache est formée d'un corps solidaire et dans le prolongement de la tête de la vis pédiculaire (ou extrémité supérieure du côté opposé ou filetage de la vis), sur lequel est fixée la bague.

Plus précisément, la bague solidaire de l'attache est par exemple au départ indépendante de celle-ci avec laquelle elle est fixée ensuite de façon amovible ou inamovible.

Dans des modes de réalisation avantageux, on a par ailleurs et/ou en outre recours à l'une et/ou à l'autre des dispositions suivantes :
- les moyens de blocage sont mobiles en rotation par rapport à la bague, ce qui permet de s'affranchir du vrillage de la bande ;

Avantageusement ils sont insérés dans un orifice cylindrique latéral de la bague (déporté latéralement par rapport à la vis et/ou au corps de l'attache) avec lequel ils coopèrent à frottement doux.

Les moyens de blocage sont alors libres en rotation autour de l'axe de l'orifice, avantageusement parallèle à l'axe de la vis et du corps.
- le corps est en forme de coupelle agencé pour être traversé latéralement par la tige perpendiculairement à la vis pédiculaire et muni d'un taraudage interne dans sa partie supérieure, de fixation de la tige par le biais d'une vis de compression, et la bague comprend un premier orifice traversant clipsable ou fixable par déformation sur la périphérie supérieure de l'attache et un second orifice latéral traversant de support des moyens de blocage ;
- l'attache comprend une partie supérieure cylindrique ou en portion de cylindre d'un premier diamètre, et le premier orifice est cylindrique de diamètre complémentaire au premier diamètre, avec laquelle il est agencé pour coopérer à frottement avant vissage de la vis de compression et pour se bloquer par légère expansion de l'attache lors du serrage.

L'expansion est par exemple de 0,2 mm en diamètre, ce qui est suffisant pour bloquer tout mouvement de la bague sur l'attache avec laquelle elle est en contact cylindre/cylindre.
- l'attache comprend une partie supérieure tronconique, le premier orifice étant tronconique de forme complémentaire à ladite partie supérieure avec laquelle il coopère à frottement, et comprend au moins un ergot de blocage vertical en partie basse ;
- l'attache comprend une partie supérieure munie d'une gorge ou d'une nervure de rétention et le premier orifice traversant de la bague est muni d'une nervure ou d'une gorge de forme correspondante agencée pour être encastrée en force l'une dans l'autre ;
- les moyens de blocage comprennent un corps de base rigide comportant un orifice traversant, et une pièce de maintien insérable dans ledit orifice, la pièce de maintien étant au moins en partie en forme de coin écrasable comprenant un alésage central de passage des portions d'extrémité en vis à vis de la bande, ledit alésage étant de section transversale déformable entre une première section de passage libre des portions d'extrémité lorsque la pièce n'a pas été insérée dans l'orifice et une deuxième section de blocage desdites portions d'extrémité en compression lorsque la pièce est entièrement ou sensiblement entièrement insérée dans l'orifice.

Avantageusement le blocage se fait par des dents antiretours, par exemple en forme de V.
- le second orifice de la bague comprend une nervure ou une gorge de rétention et la pièce de maintien comprend une gorge ou nervure complémentaire agencée pour être clipsée puis bloquée verticalement vers le haut avec la nervure ou gorge du second orifice de la bague.

Une telle disposition autorise la rotation de l'une par rapport à l'autre ;
- la vis pédiculaire est à tête ronde ou oblongue montée pivotante en rotation dans la partie inférieure de l'attache et l'attache comporte une structure de jonction entre la paroi cylindrique de la tige et ladite tête de vis.

La structure de jonction épouse la forme de la tige d'un côté et celle de la tête de l'autre.

L'invention concerne également un système comprenant une tige et au moins un dispositif tel que décrit ci-dessus.

Un procédé non revendiqué de fixation d'une tige sur une vertèbre est décrit dans lequel on visse dans la vertèbre une vis pédiculaire munie d'une attache sur la tige, on glisse la tige au travers de l'attache et on visse une vis de compression sur la tige au travers de la partie supérieure de l'attache, caractérisé en ce que, avant de visser la vis de compression, on passe une bande souple autour de ladite vertèbre, on clipse sur la périphérie supérieure de l'attache un premier orifice traversant d'une bague, clipsable ou fixable par déformation sur ladite périphérie supérieure, ladite bague comprenant un second orifice latéral traversant de support de moyens de blocage réglables de la bande souple, lesdits moyens de blocage étant mobiles en rotation par rapport à la bague et/ou l'attache de fixation, on place la bande souple sur la vertèbre et dans les moyens de blocage, on commence à mettre ladite bande souple en tension, la libre rotation permettant de limiter les contraintes de vrillage de la bande puis on met le tout en tension avant de bloquer définitivement la vis de compression et la bande par rapport à l'attache.

Avantageusement les moyens de blocage comprennent un corps de base rigide comportant un orifice traversant, et une pièce de maintien insérable dans ledit orifice, la pièce de maintien étant au moins en partie en forme de coin écrasable comprenant un alésage central de passage des portions d'extrémité en vis à vis de la bande, ledit alésage étant de section transversale déformable entre une première section de passage libre des portions d'extrémité lorsque la pièce n'a pas été insérée dans l'orifice et une deuxième section de blocage desdites portions d'extrémité en compression lorsque la pièce est entièrement ou sensiblement entièrement insérée dans l'orifice, on insère la bande dans le corps de base et la pièce de maintien et après mise en tension et pour bloquer la bande en déplacement, on insère entièrement la pièce dans le corps.

L'invention sera mieux comprise à la lecture de la description qui suit de modes de réalisation donnés ci-après à titre d'exemples non limitatifs. La description se réfère aux dessins qui l'accompagnent dans lesquels :
La figure 1 est une vue en perspective axionométrique d'un dispositif et/ou système de fixation selon un premier mode de réalisation de l'invention.
La figure 1A est une vue en coupe selon un I_{A} - I_{A} de la figure 1 sans la bande souple.
La figure 2 est une vue en perspective de la bague du type de celle utilisée dans le dispositif de la figure 1.
La figure 2A est une vue en coupe selon II_{A} - II_{A} de la figure 2.
Les figures 3A et 3B sont des vues en perspective des moyens de blocage selon un mode de réalisation de l'invention, avant insertion (figure 3A) et après insertion complète (figure 3B) de la pièce de maintien dans le corps de base.
La figure 4 est une vue en coupe longitudinale d'un dispositif et/ou système selon un autre mode de réalisation de l'invention.
La figure 5 est une vue en perspective de la bague utilisée dans le dispositif de la figure 4.
La figure 5A est une vue en coupe selon V_{A} - V_{A} de la bague de la figure 5.
La figure 6 est une vue partielle en perspective d'un autre mode de réalisation de la partie supérieure d'un dispositif selon l'invention.
La figure 6A est une vue en perspective et en coupe selon VI_{A} - VI_{A} de la figure 6.
La figure 7 est une vue agrandie en coupe de la bague des figures 6 et 6A.
La figure 8 est une vue en perspective d'un système comprenant un dispositif selon un mode de réalisation de l'invention en référence à la figure 1 et une tige en position sur les vertèbres.

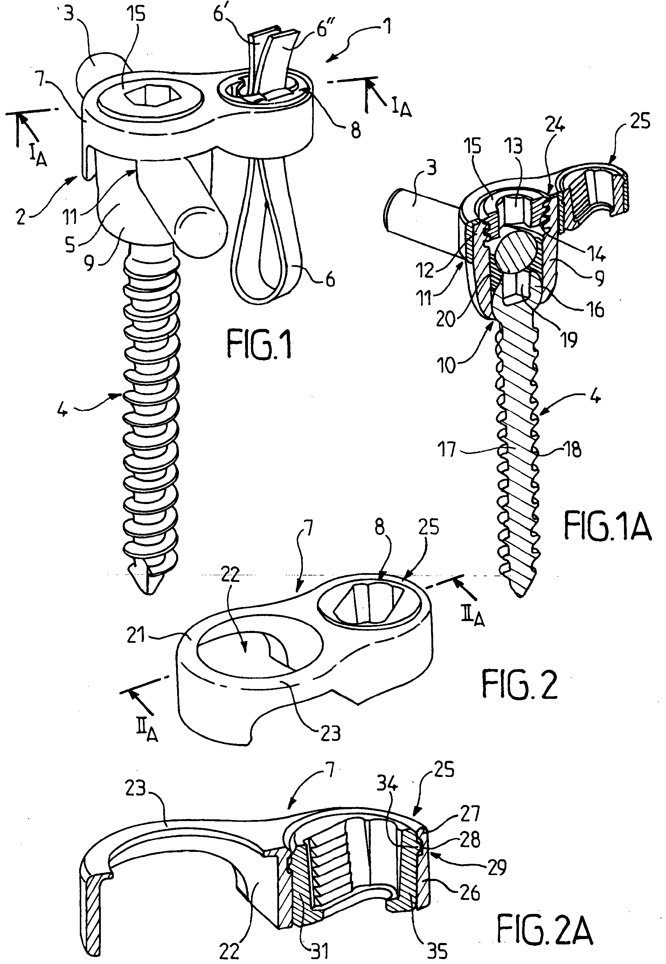

La figure 1 montre un système 1 comprenant un dispositif 2 de fixation d'une vertèbre (non représentée) sur une tige 3, comportant une vis pédiculaire 4 et une attache 5 de fixation de la vis sur la tige.

Le dispositif 2 comporte une bande 6 souple et plate par exemple en polyester tressé, par exemple de 1 à 3 mm d'épaisseur et de 6 mm de large et présente une boucle de fixation sur la vertèbre, par exemple autour d'une de ses branches transverses ou épineuses (non représentée).

La boucle est formée par le rapprochement des portions d'extrémité 6' et 6" de la bande.

Le dispositif 2 comporte une bague 7 solidaire de l'attache 5 et des moyens 8 de blocage réglable de la bande souple par rapport à l'attache 5, par blocage de ces deux portions d'extrémité 6, 6' comme cela va être décrit plus précisément ci-après.

En référence également à la figure 1A, l'attache 5 comprend un corps 9 sensiblement cylindrique en forme de coupelle évidée percée en sa partie inférieure ou fond 10 pour laisser passer la vis pédiculaire 4, et comporte deux encoches latérales 11, ouvertes vers le haut, de passage latéral de la tige 3 qui vient s'y encastrer.

Le corps 9 par exemple en titane, est cylindrique dans sa partie supérieure 12 et évasé vers l'intérieur dans sa partie inférieure.

Le corps 9 comprend un évidement 13 cylindrique de passage, et de blocage dans le sens axial de la vis. La partie supérieure 12 comprend un taraudage interne 14 de fixation de la tige, introduite dans les encoches latérales 11, par une vis 15 de compression qui vient donc comprimer la tige sur la tête 16 de la vis pédiculaire 4.

Celle-ci comprend un corps 17 de vissage muni d'un pas de vis 18 du type connu en lui-même.

La tête 16 est munie d'une encoche 19 de vissage par un outil. Elle est partiellement sphérique et vient s'appuyer sur le fond 10 de la coupelle du corps 9 de l'attache avec qui elle coopère à frottement en rotation et en butée longitudinale.

Après vissage de la vis pédiculaire 4 préalablement introduite dans l'attache 5, et après introduction au-dessus de la tête de vis d'une pièce 20 de jonction/guidage/soutien en forme d'anneau d'un côté (tête de vis) et de demi cylindre (tige) de l'autre, puis après introduction de la tige 3, on met en place la bague 7.

La bague 7 est une pièce par exemple en titane, en forme de haricot aplati, de section longitudinale horizontale en forme de 8 dont les boucles ne sont pas de mêmes dimensions.

Elle comprend (cf. figure 2 et 2A) une première portion sensiblement cylindrique 21 munie d'un alésage cylindrique interne 22 ou premier orifice comprenant une partie supérieure 23 rétrécie formant cornière de blocage en déplacement axial avec le dessus 24 de l'attache 5.

L'alésage interne 22 coopère à frottement en contact cylindre/cylindre avec la surface externe de la partie supérieure cylindrique 12 de l'attache.

Une fois la bague insérée sur la partie supérieure de l'attache en frottement, la vis 15 de serrage en compression est vissée.

De par ce vissage, la tête de la vis 15 étant conçue pour, on réalise une légère expansion du volume qu'elle occupe lors du serrage, par exemple de l'ordre de 0,2 mm, qui déforme en compression latérale l'alésage de l'attache, ce qui bloque tout mouvement de la bague par rapport à l'attache.

La bague 7 comprend latéralement par rapport au premier orifice un second orifice 25, traversant, de support des moyens 8 de blocage.

Le second orifice 25 (voir figure 2A) est cylindrique d'axe parallèle ou sensiblement parallèle à celui du premier orifice et/ou de la vis pédiculaire. Il comprend une portion inférieure 26 et une portion supérieure 27 munie d'une rainure 28 à sa partie d'extrémité supérieure 29.

Cette rainure est agencée pour coopérer avec une nervure complémentaire appartenant aux moyens 8 de blocage qui vont maintenant être décrits.

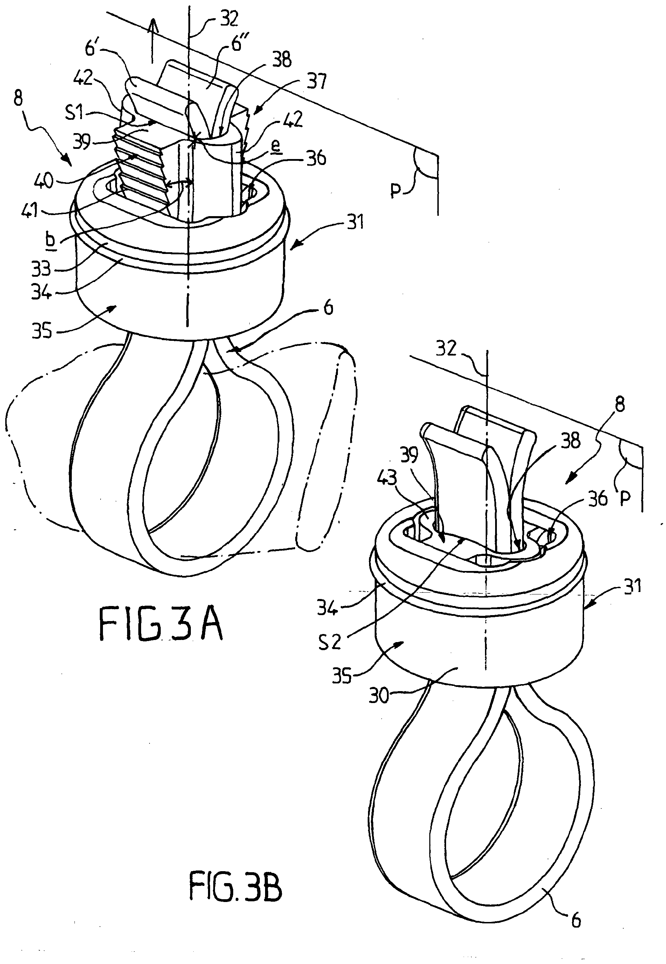

En références aux figures 3A et 3B les moyens 8 comprennent un corps 31 de base rigide, par exemple en titane (il peut également être en matière plastique rigide).

Le corps 31 est allongé autour d'un axe 32. Il est cylindrique et comprend à sa partie supérieure 33 la nervure complémentaire 34 agencée pour permettre un blocage verticalement vers le haut avec le second orifice de la bague, tout en autorisant une rotation de l'un par rapport à l'autre.

Sa partie inférieure 35 est cylindrique et coopère à frottement (autorisant une rotation autour de l'axe 32) avec la portion inférieure 26 du second orifice de la bague.

Le corps 31 est muni d'un orifice traversant 36 de section sensiblement oblongue.

Les moyens 8 comprennent de plus une pièce 37 de maintien de la bande 6, par exemple en matière plastique, au moins en partie en forme de coin, c'est à dire de forme prismatique ou sensiblement prismatique.

La pièce 37 est percée d'un alésage 38 de passage des extrémités 6', 6" de la bande, de section transversale déformable entre une première section S1 de passage libre des portions d'extrémité lorsque la pièce 37 n'est pas insérée en compression réelle dans l'orifice (figure 3A) et une section S₂, rétrécie, de blocage des portions d'extrémité en compression lorsque la pièce est entièrement insérée dans l'orifice 36 (figure 3B).

Plus précisément, la pièce 37 présente une première partie centrale 39 de section horizontale rectangulaire ou sensiblement rectangulaire décroissante, et de section verticale longitudinale de forme trapézoïdale ou sensiblement trapézoïdale isocèle à parois 40 symétriques par rapport à un plan longitudinal P, à la périphérie dentelée régulièrement extérieurement (dents 41).

Les dents 41 (par exemple au nombre de sept) sont formées par des arêtes longitudinales en triangle, dont les sommets sont dirigés vers le haut ou sont horizontaux, à angle aigu (en écaille).

Les dents inférieures ou la dent inférieure de la pièce et les ou la dent(s) supérieure(s) de l'orifice du corps, forment un moyen de pré-connexion de l'un par rapport à l'autre par encliquetage.

La pièce 37 présente de plus deux parties d'extrémités 42 en forme de demi cylindre ou sensiblement de demi-cylindre présentant des parois de section transversale ovale, symétriques par rapport au plan perpendiculaire au plan longitudinal P, dont l'ovalité est dirigée vers l'extérieur et est agencée, comme on va le voir pour coopérer avec des parois en vis à vis de l'orifice 36.

La section trapézoïdale présente un angle à la base b (par rapport à la petite base du trapèze).

L'alésage 38 présente donc une section transversale horizontale de forme oblongue, en langue de chat qui va se déformer entre une forme de section S1 (figure 3A) et une forme à milieu rétréci de section S2 (figure 3B).

Dans le mode de réalisation plus particulièrement décrit ici la pièce 37 présente donc un noyau 43 évidé par l'alésage 38 formé par la première partie centrale 39 et les parties d'extrémités 42.

Il présente les parois principales opposées 40 dont la face externe est en biais et forme le coin relié par les deux parois arrondies d'extrémité 42 agencées pour coopérer à frottement avec l'orifice qui sera décrit ci-après.

L'épaisseur e des jonctions 22 entre parois principales et parois d'extrémité 42 est plus faible (par exemple deux fois moins épaisse) pour autoriser la déformation par écrasement dans le sens transversal sans déformation dans le sens longitudinal lors de l'insertion de la pièce dans l'orifice (cf. figure 3B).

Dans la suite de la description on utilisera les mêmes numéros de référence pour désigner des éléments identiques ou similaires.

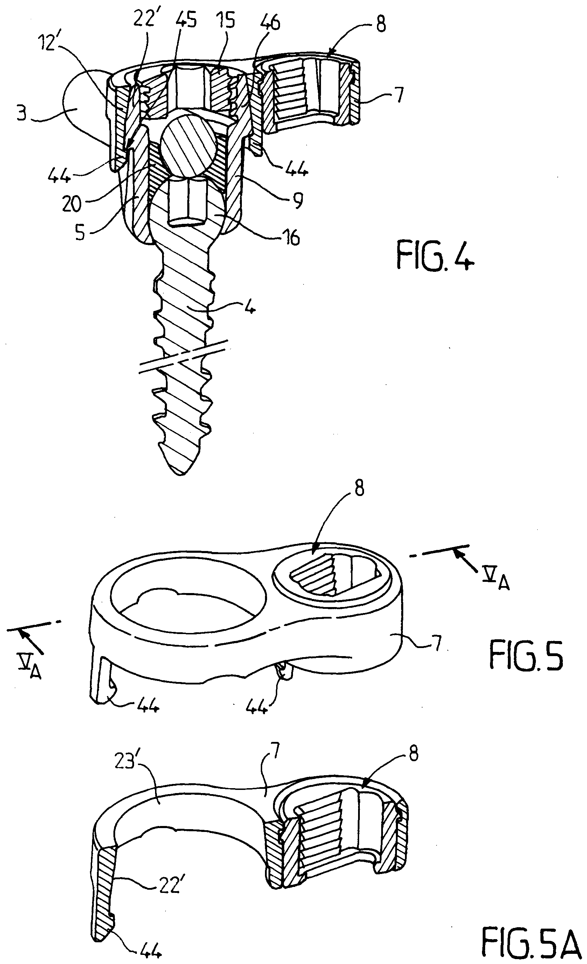

La figure 4 montre un système (sans bande) avec vis pédiculaire 4, pièce de jonction/guidage 20, tige 3, vis 15 de compression et partie supérieure 12' du corps 9 différente de celle décrite en référence aux figures 1 et 1A.

Ici la jonction entre la paroi externe de la partie supérieure 12' de l'attache et la paroi interne de l'alésage du premier orifice 22' est tronconique (petite base vers le haut), à frottement par exemple de type cône morse. La bague 7 (cf. figure 5 et 5A) comprend deux ergots 44 en partie inférieure de l'orifice, déformables, qui viennent coopérer avec la face inférieure 45 d'une partie inférieure en excroissance 46 de la partie supérieure 12' avec laquelle ils s'encliquent, fixant la bague sur l'attache.

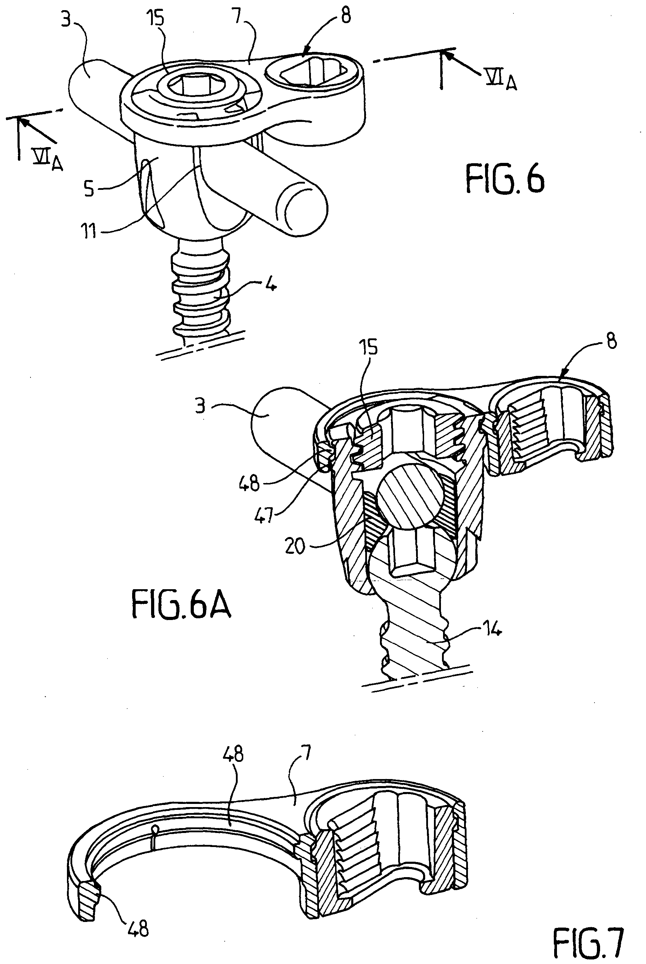

Les figures 6, 6A et 7 montrent un autre mode de réalisation d'attache et de bague selon l'invention.

Ici l'attache 5 comprend une partie supérieure munie d'une gorge de rétention 47 et le premier orifice 22 est muni d'une nervure 48 de forme correspondante agencée pour être encastrée à force l'une dans l'autre.

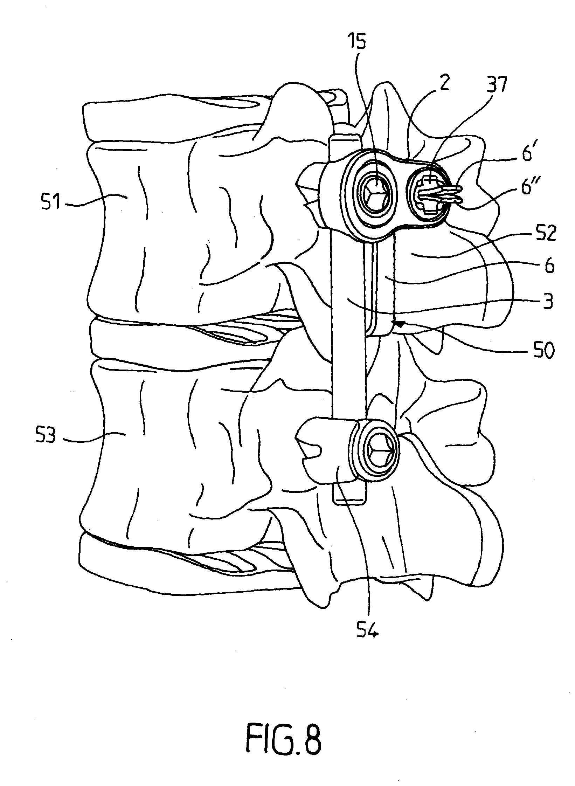

La figure 8 est une vue en perspective d'un système 50 comprenant un dispositif 2 selon le mode de réalisation de la figure 1.

Le dispositif 2 est fixé dans une vertèbre 51 de qualité médiocre, et dont la fixation est assumée d'une part par la vis pédiculaire dont la tête est maintenue sur la tige 3 par vissage de la vis de compression 15 et d'autre part par la bande 6 passée autour de l'épiphyse 52 de la vertèbre 51.

La tige 3 est par ailleurs fixée de façon connue sur la vertèbre d'à côté 53 par un dispositif 54 à vis pédiculaire, de façon connue en elle-même.

On va maintenant décrire en référence aux figures 1, 3A, 3B et 8 la mise en œuvre du système plus particulièrement décrit ici.

Après avoir vissé les deux vis pédiculaires correspondant aux dispositifs 2 et 54 munis de leurs attaches respectives, dans les vertèbres adjacentes 51, 52, le chirurgien insère la tige 3 dans les fentes 11 des têtes d'attache, puis clipse la bague munie des moyens de blocage dans lesquels il a posé un premier brin 6' de la bande 6.

Il passe alors la bande souple (brin 6") autour de la vertèbre et l'introduit dans les moyens de blocage (c'est à dire dans la pièce de maintien 37) puis met la bande en tension.

La libre rotation de la pièce 37 permet d'éviter le vrillage de la bande.

Il visse alors la vis 15 de pression de la tige, la tige et la bague étant alors bloquées en mouvement.

Puis il finit de mettre en tension la bande 37.

Une fois la tension recherchée obtenue, la pièce 37 est complètement introduite dans le corps 31, bloquant définitivement par coincement les deux brins 6' et 6".

Comme il va de soi et comme il résulte également de ce qui précède, la présente invention n'est pas limitée aux modes de réalisation plus particulièrement décrits. Elle en embrasse au contraire toutes les variantes et notamment celles où les moyens de blocage comprennent de plus des nervures de blocage en excroissance à l'intérieur de l'alésage traversant de blocage des brins 6' et 6".

## Revendications

1. Dispositif (2) de fixation d'une vertèbre rachidienne sur une tige (3), comportant une vis pédiculaire (4), une attache (5) de fixation de la vis sur la tige, et une bande (6) souple de liaison avec la vertèbre, **caractérisé en ce qu'**il comporte une bague (7) rapportée solidaire de l'attache, et des moyens (8), solidaires de ladite bague, de blocage réglable de la bande (6) souple par rapport à l'attache (5) de fixation et **en ce que** les moyens (8) de blocage comprennent un corps (31) de base rigide comportant un orifice traversant (36), et une pièce (37) de maintien insérable dans ledit orifice, la pièce de maintien étant au moins en partie en forme de coin écrasable comprenant un alésage (38) central de passage des portions (6', 6") d'extrémité en vis à vis de la bande, ledit alésage étant de section transversale déformable entre une première section (S1) de passage libre des portions d'extrémité lorsque la pièce n'a pas été insérée dans l'orifice et une deuxième section (S2) de blocage desdites portions d'extrémité en compression lorsque la pièce est entièrement ou sensiblement entièrement insérée dans l'orifice.

2. Dispositif selon la revendication 1, **caractérisé en ce que** l'attache est formée d'un corps (9) solidaire et dans le prolongement de la tête de la vis pédiculaire, sur lequel est fixée la bague.

3. Dispositif selon l'une quelconque des revendications 1 ou 2, **caractérisé en ce que** les moyens (8) de blocage sont mobiles en rotation par rapport à la bague (7), ce qui permet de s'affranchir du vrillage de la bande.

4. Dispositif selon la revendication 3, **caractérisé en ce que** les moyens (8) de blocage sont insérés dans un orifice cylindrique latéral de la bague déporté par rapport à la vis avec lequel ils coopèrent à frottement doux.

5. Dispositif selon l'une quelconque des revendications 2 à 4, **caractérisé en ce que** le corps (9) de l'attache est en forme de coupelle agencé pour être traversé latéralement par la tige (3) perpendiculairement à la vis pédiculaire (4) et muni d'un taraudage interne (14) dans sa partie supérieure, de fixation de la tige par le biais d'une vis (15) de compression, et **en ce que** la bague comprend un premier orifice (22) traversant clipsable par déformation sur la périphérie supérieure de l'attache et un second orifice (25) latéral traversant de support des moyens de blocage.

6. Dispositif selon l'une quelconque des revendications 1 à 4, **caractérisé en ce que** l'attache comprend une partie supérieure cylindrique ou en portion de cylindre d'un premier diamètre, et le premier orifice est cylindrique de diamètre complémentaire au premier diamètre, avec laquelle il est agencé pour coopérer à frottement avant vissage de la vis de compression et pour se bloquer par légère expansion de l'attache lors du serrage.

7. Dispositif selon l'une quelconque des revendications 1 à 4, **caractérisé en ce que** l'attache comprend une partie supérieure tronconique, le premier orifice étant tronconique de forme complémentaire à ladite partie supérieure avec laquelle il coopère à frottement, et comprend au moins un ergot de blocage vertical en partie basse.

8. Dispositif selon l'une quelconque des revendications 1 à 4, **caractérisé en ce que** l'attache comprend une partie supérieure munie d'une gorge ou d'une nervure de rétention et le premier orifice traversant de la bague est muni d'une nervure ou d'une gorge de forme correspondante agencée pour être encastrée en force l'une dans l'autre.

9. Dispositif selon la revendication 8, **caractérisé en ce que** le second orifice (25) de la bague (7) comprend une nervure ou une gorge de rétention et la pièce de maintien comprend une gorge ou une nervure complémentaire agencée pour être clipsée puis bloquée verticalement vers le haut avec la nervure ou gorge du second orifice de la bague.

10. Dispositif selon l'une quelconque des revendications précédentes, **caractérisé en ce que** la vis pédiculaire (4) est à tête ronde ou oblongue (16) montée pivotante en rotation dans la partie inférieure de l'attache et **en ce que** l'attache comporte une structure (20) de jonction entre la paroi cylindrique de la tige et ladite tête de vis.

11. Système de fixation d'une vertèbre rachidienne comportant une tige et au moins un dispositif selon l'une quelconque des revendications précédentes.

## Patentansprüche

1. Vorrichtung (2) zur Befestigung eines Wirbelsäulenwirbels an einem Stift (3), umfassend eine Pedikelschraube (4), ein Element (5) zur Befestigung der Schraube an dem Stift und ein flexibles Band (6) zur Verbindung mit dem Wirbel, **dadurch gekennzeichnet, dass** sie einen mit dem Element fest verbundenen angeformten Ring (7) und mit dem Ring fest verbundene Mittel (8) zur einstellbaren Arretierung des flexiblen Bands (6) in Bezug auf das Befestigungselement (5) umfasst und dass die Arretierungsmittel (8) einen starren Grundkörper (31) mit einer Durchgangsöffnung (36) und ein in die Öffnung einsetzbare Haltestück (37) umfassen, wobei das Haltestück mindestens teilweise als zusammendrückbarer Keil mit einer mittigen Bohrung (38) für die Durchführung der einander gegenüberliegenden Endabschnitte (6', 6") des Bands ausgeformt ist, wobei die Bohrung einen Querschnitt aufweist, der zwischen einem ersten Bereich (S1) für die freie Durchführung der Endabschnitt, wenn das Stück nicht in die Öffnung eingesetzt worden ist, und einem zweiten Bereich (S2) für die Arretierung der zusammengedrückten Endabschnitte, wenn das Stück vollständig oder im Wesentlichen vollständig in die Öffnung eingesetzt ist, verformbar ist.

2. Vorrichtung nach Anspruch 1, **dadurch gekennzeichnet, dass** das Element durch einen fest verbundenen, in der Verlängerung des Kopfes der Pedikelschraube angeordneten Körper (9) gebildet ist, an dem der Ring befestigt ist.

3. Vorrichtung nach einem beliebigen der Ansprüche 1 oder 2, **dadurch gekennzeichnet, dass** die Arretierungsmittel (8) in Bezug auf den Ring (7) drehbeweglich sind, wodurch das Band sich nicht verdrehen kann.

4. Vorrichtung nach Anspruch 3, **dadurch gekennzeichnet, dass** die Arretierungsmittel (8) in eine seitliche zylindrische, in Bezug auf die Schraube versetzte Öffnung des Rings eingesetzt sind, mit der sie leicht reibschlüssig zusammenwirken.

5. Vorrichtung nach einem beliebigen der Ansprüche 2 bis 4, **dadurch gekennzeichnet, dass** der Körper (9) des Elements die Form einer Schale aufweist, welche angeordnet ist, um seitlich von dem Stift (3) senkrecht zu der Pedikelschraube (4) durchquert zu werden und welche in ihrem oberen Bereich mit einem Innengewinde (14) für die Befestigung des Stifts durch eine Kompressionsschraube (15) versehen ist, und dass der Ring eine erste durch Verformung auf den oberen Umfang des Elements aufclipsbare Durchgangsöffnung (22) und eine zweite seitliche Durchgangsöffnung (25) zum Stützen der Arretierungsmittel umfasst.

6. Vorrichtung nach einem beliebigen der Ansprüche 1 bis 4, **dadurch gekennzeichnet, dass** das Element einen oberen zylindrischen oder zylinderabschnittsförmigen Bereich mit einem ersten Durchmesser umfasst und die erste Öffnung zylindrisch mit einem zum ersten Durchmesser komplementären Durchmesser ist, mit dem sie angeordnet ist, um vor Eindrehen der Kompressionsschraube reibschlüssig zusammenzuwirken und um durch leichte Ausweitung des Elements beim Anziehen zu blockieren.

7. Vorrichtung nach einem beliebigen der Ansprüche 1 bis 4, **dadurch gekennzeichnet, dass** das Element einen kegelstumpfförmigen oberen Bereich umfasst, wobei die erste Öffnung kegelstumpfförmig komplementär zum oberen Bereich geformt ist, mit dem sie reibschlüssig zusammenwirkt, und mindestens eine senkrechte Arretierungsnase im unteren Bereich aufweist.

8. Vorrichtung nach einem beliebigen der Ansprüche 1 bis 4, **dadurch gekennzeichnet, dass** das Element einen oberen Bereich mit einer Rückhaltenut bzw. -rippe umfasst und die erste Durchgangsöffnung des Rings mit einer Nut bzw. Rippe mit korrespondierender Form versehen ist, welche angeordnet ist, um mit Kraftschluss ineinander gesteckt zu werden.

9. Vorrichtung nach Anspruch 8, **dadurch gekennzeichnet, dass** die zweite Öffnung (25) des Rings (7) eine Rückhalterippe bzw. - nut aufweist und das Haltestück eine komplementäre Nut bzw. Rippe aufweist, welche angeordnet ist, um mit der Rippe bzw. Nut der zweiten Öffnung des Rings in Eingriff zu gelangen und dann senkrecht nach oben arretiert zu werden.

10. Vorrichtung nach einem beliebigen der vorhergehenden Ansprüche, **dadurch gekennzeichnet, dass** die Pedikelschraube (4) einen runden oder länglichen Kopf (16) aufweist, welcher drehkippbar im unteren Bereich des Elements montiert ist, und dass das Element eine Verbindungsstruktur (20) zwischen der zylindrischen Wand des Stifts und dem Schraubenkopf aufweist.

11. System zur Befestigung eines Wirbelsäulenwirbels mit einem Stift und mindestens einer Vorrichtung nach einem beliebigen der vorhergehenden Ansprüche.

## Claims

1. Device (2) for fixing a spinal vertebra to a rod (3), having a pedicle screw (4), a lug (5) for fixing the screw to the rod, and a flexible band (6) for connection to the vertebra, **characterized in that** it has an attached ring (7) rigidly connected to the lug, and means (8) rigidly connected to said ring for adjustably blocking the flexible band (6) with respect to the fixing lug (5), and **in that** the blocking means (8) comprise a rigid base body (31) with a through-orifice (36), and a holding component (37) that is insertable into said orifice, the holding component being at least partially in the shape of a squeezable wedge comprising a central bore (38) for passage of the opposite end portions (6', 6") of the band, said bore having a cross section that is deformable between a first cross section (S1) for free passage of the end portions when the component has not been inserted into the orifice, and a second cross section (S2) for blocking said end portions by compression when the component is entirely or substantially entirely inserted into the orifice.

2. Device according to Claim 1, **characterized in that** the lug is formed by a body (9) rigidly connected to and forming a continuation of the head of the pedicle screw to which the ring is fixed.

3. Device according to either of Claims 1 and 2, **characterized in that** the blocking means (8) are movable in rotation with respect to the ring (7), whereby it is possible to avoid twisting of the band.

4. Device according to Claim 3, **characterized in that** the blocking means (8) are inserted into a lateral cylindrical orifice of the ring, which orifice is offset in relation to the screw and with which said means cooperate by slight friction.

5. Device according to any one of Claims 2 to 4, **characterized in that** the body (9) of the lug has a cup shape, designed to be traversed laterally by the rod (3) perpendicularly with respect to the pedicle screw (4), and is provided with an internal thread (14) in its upper part, for fixing the rod by way of a compression screw (15), and **in that** the ring comprises a first through-orifice (22) which can be clipped by deformation onto the upper periphery of the lug, and a second lateral through-orifice (25) for supporting the blocking means.

6. Device according to any one of Claims 1 to 4, **characterized in that** the lug comprises an upper part which is cylindrical or shaped as a portion of a cylinder with a first diameter, and the first orifice is cylindrical with a diameter matching the first diameter, with which it is designed to cooperate by friction, before the compression screw is screwed in, and to become blocked by slight expansion of the lug during the clamping.

7. Device according to any one of Claims 1 to 4, **characterized in that** the lug comprises a frustoconical upper part, the first orifice having a frustoconical shape matching said upper part with which it cooperates by friction, and comprises at least one vertical blocking stub in the bottom portion.

8. Device according to any one of Claims 1 to 4, **characterized in that** the lug comprises an upper part provided with a retaining groove or rib, and the first through-orifice of the ring is provided with a rib or groove of corresponding shape designed to be engaged with force one inside the other.

9. Device according to Claim 8, **characterized in that** the second orifice (25) of the ring (7) comprises a retaining rib or groove, and the holding component comprises a complementary groove or rib designed to be clipped then blocked vertically upward with the rib or groove of the second orifice of the ring.

10. Device according to any one of the preceding claims, **characterized in that** the pedicle screw (4) has a round or oblong head (16) mounted pivotably in rotation in the lower part of the lug, and **in that** the lug has a joining structure (20) between the cylindrical wall of the rod and said screw head.

11. System for fixing a spinal vertebra, having a rod and at least one device according to any one of the preceding claims.
